# EUROPEAN PATENT APPLICATION

(11) **EP 1 857 542 A1**
(43) Date of publication of application: **21.11.2007**
(21) Application number: 06714933.6
(22) Date of filing: 22.02.2006
(51) Int. Cl.: C12N 5/00, C12N 1/00, C12N 5/06

(54) **METHOD FOR IN VITRO AMPLIFICATION OF ADULT STEM CELLS**

(30) Priority: 28.02.2005 JP 2005052944
(71) Applicant: Foundation for Biomedical Research and Innovation, Chuo-ku Kobe-shi Hyogo 6500047 (JP); TOKAI UNIVERSITY EDUCATIONAL SYSTEM, Shibuya-ku Tokyo 1510063 (JP)
(72) Inventor: ASAHARA, Takayuki, Institute of Biomedical R & I, Kobe-shi, Hyogo, 6500047 (JP); ISHIKAWA, Tetsuya, c/o Tokai University, Kanagawa, 259-1193 (JP)
(74) Representative: Duckworth, Timothy John
(86) International application number: PCT/JP2006/303810
(87) International publication number: WO 2006/093172

(57) **Abstract**

The present invention provides a method of efficiently expanding non-adhesive adult stem cells in vitro. More specifically, the present invention provides a method of expanding non-adhesive adult stem cells, comprising culturing non-adhesive adult stem cells in the co-presence of adhesive feeder cells and non-adhesive non-stem cells in serum-free medium, wherein the ratio of non-adhesive adult stem cell count to total cell count in the medium is kept low; non-adhesive adult stem cells obtained by the expansion method and differentiated cells thereof, and a composition comprising them; and a serum-free medium comprising a specified factor, and a kit for its preparation and the like.

## Description

### Technical Field

The present invention relates to a method of expanding adult stem cells in vitro. More specifically, the present invention relates to a method of expanding adult stem cells more than several hundred fold under serum-free culture conditions, various cells prepared by the method, and the like.

### Background Art

Currently, about 100,000 patients per year, including latent patients, are suffering from symptoms of lower limb ischemia due to Buerger disease, chronic obstructive arteriosclerosis and the like, and there are about 1,100,000 patients with ischemic heart diseases, including myocardial infarction, which reportedly affects about 200,000 people per year. For these diseases, cell transplantation therapy, gene therapy, G-CSF therapy, bypass surgery and PTCA therapy are performed.

As the cell transplantation therapy for ischemic heart diseases, bone-marrow momonuclear cell transplantation therapy and angiogenesis therapy using vascular endothelial progenitor cells (hereinafter also abbreviated as EPC) obtained by collecting peripheral blood stem cells are known. However, when peripheral blood stem cells are used, theoretically more than about 10 L of peripheral blood is required for the purpose of apheresis and the like, posing a major burden on the patient. Furthermore, in bone-marrow momonuclear cells transplantation therapy, it is necessary to collect more than 500 mL of bone marrow fluid under general anesthesia, again posing a major burden on the patient, so that repeated treatment has been difficult.

For these reasons, there has been a demand for a method of cell culture in vitro that enables the obtainment of larger amounts of stem cells that can be used for cell transplantation, without posing a burden on the patient. For example, cells could be expanded about 4 fold in ex vivo cultivation of hematopoietic stem cells by a cultivation method using serum (Ueda et al., The Journal of Clinical Investigation vol.105, No.7, pp.1013-1021 (2000)), and about 10 fold in ex vivo cultivation of hematopoietic stem cells using mouse cells as the feeder cells (Kawada et al., Experimental Hematology vol.27, pp.904-915 (1999)), respectively, but these results cannot be said to have been clinically satisfactory.

### Disclosure of the Invention

The present invention is intended to provide a method of efficiently expanding non-adhesive adult stem cells in vitro, and also intended to provide various cells obtained by inducing differentiation from stem cells obtained by the cultivation method and the like.

In view of the above-described problems, the present inventors investigated cultivation conditions that allow the expansion of non-adhesive adult stem cells in vitro, and that ensure higher expansion efficiency than expected conventionally. As a result, the present inventors unexpectedly found that in culturing non-adhesive adult stem cells in the co-presence of adhesive feeder cells in a serum-free medium supplemented with specified factors and the like, the expansion efficiency was remarkably improved by keeping the ratio of non-adhesive adult stem cells low, and the like, confirmed that the expanded cells could be induced to differentiate into desired cells by adding specified factors and the like, and developed the present invention.

Accordingly, the present invention provides:
[1] A method of expanding non-adhesive adult stem cells, comprising culturing the non-adhesive adult stem cells in the co-presence of adhesive feeder cells and non-adhesive non-stem cells in serum-free medium, wherein the ratio of non-adhesive adult stem cell count to total non-adhesive cell count in the medium is not more than 40%;
[2] the method according to [1] above, comprising adding a biological sample comprising non-adhesive adult stem cells, adhesive feeder cells and non-adhesive non-stem cells to serum-free medium without a cell fractionation treatment utilizing a cell surface marker, and then culturing the non-adhesive adult stem cells in the serum-free medium in the co-presence of the adhesive feeder cells and the non-adhesive non-stem cells;
[3] the method according to [1] above, further comprising adjusting the ratio of non-adhesive adult stem cells to total non-adhesive cell count before the start of cultivation;
[4] the method according to [1] above, further comprising confirming the expansion of non-adhesive adult stem cells, or isolating the non-adhesive adult stem cells expanded;
[5] the method according to [1] above, wherein the ratio of non-adhesive adult stem cell count to total non-adhesive cell count in the medium at the time of start of cultivation is not more than 20%;
[6] the method according to [1] above, wherein the non-adhesive adult stem cells comprise CD34 and/or CD133 positive cells;
[7] the method according to [1] above, wherein the non-adhesive adult stem cells are momonuclear cells;
[8] the method according to [1] above, wherein the adhesive feeder cells are momonuclear cells;
[9] the method according to [1] above, wherein the non-adhesive adult stem cells and/or adhesive feeder cells are derived from bone marrow, cord blood or peripheral blood;
[10] the method according to [1] above, wherein the non-adhesive adult stem cells and adhesive feeder cells are derived from the same animal species;
[11] the method according to [2] above, wherein the biological sample is selected from the group consisting of bone marrow fluid, cord blood and peripheral blood;
[12] the method according to [2] above, wherein the biological sample is derived from a human;
[13] the method according to [1] above, wherein the serum-free medium comprises interleukin 3 and a fatty acid;
[14] the method according to [1] above, further comprising differentiating non-adhesive adult stem cells expanded by culturing non-adhesive adult stem cells to obtain differentiated cells;
[15] the method according to [14] above, wherein the differentiated cells are selected from the group consisting of vascular endothelial cells, vascular smooth muscle cells, lymphatic endothelial cells, skeletal muscle cells, myocardial cell, neural lineage cells, blood lineage cells, hepatocytes, pancreas cells, kidney cells and epithelial cells;
[16] the method according to [1] above, wherein the amount of medium per unit number of cells is not less than 0.2 ml/1.0x10⁵ cells;
[17] the method according to [1] or [16] above, wherein the cultivation is performed so that not less than 70% of cells cluster in a 3/10 volume of the medium;
[18] non-adhesive adult stem cells obtainable by the method according to [1] above;
[19] a composition comprising non-adhesive adult stem cells, and being substantially free from any biological ingredient derived from an animal heterologous to the animal from which the non-adhesive adult stem cells are derived;
[20] differentiated cells of non-adhesive adult stem cells, obtainable by the method according to [14] above;
[21] a serum-free medium comprising interleukin 3 and a fatty acid;
[22] A kit for preparing a serum-free medium comprising interleukin 3 and a fatty acid, comprising interleukin 3, the fatty acid and a serum-free medium (one or both of interleukin 3 and the fatty acid are not added to the serum-free medium).

The method of the present invention makes it possible to expand non-adhesive adult stem cells ex vivo at least 500 fold, and the expanded cells obtained can be induced to differentiate into various cells in vivo and in vitro. Furthermore, the expanded cells obtained are effective in improving bloodstream in an ischemia model and a muscular injury model. That is, by the method of the present invention, which enables obtaining large amounts of adult stem cells, it is possible to provide a therapeutic method with a less burden on the patient for reportedly about 100,000 lower limb ischemia patients, including latent patients, and for reportedly about 1,100,000 ischemic heart disease patients. Also, compared with the conventional method, which comprises collecting not less than 500 ml of bone marrow fluid under general anesthesia, the method of the present invention requires only about 1 ml of bone marrow fluid collected under local anesthesia, so that the burden on the patient can be remarkably lessened. Furthermore, because the cells after expansion can be cryopreserved, repeated treatment, dosing of 1 lot to a plurality of patients and the like are also possible.

### Best Mods for Embodying the Invention

The present invention provides a method of expanding non-adhesive (i.e., suspended) adult stem cells. The expansion method of the present invention comprises culturing non-adhesive adult stem cells in the co-presence of adhesive feeder cells and non-adhesive non-stem cells in a serum-free medium.

As used herein, "non-adhesive adult stem cells" refer to undifferentiated cells suspended in a body fluid removed from the body of an animal such as a mammal, including both cells fated to differentiate into a particular cell lineage and cells not fated to differentiate into a particular cell lineage. As the animal species from which the non-adhesive adult stem cells are derived, a bird, a mammal and the like can be mentioned, and a mammal is preferable. As examples of the mammal, rats, mice, rabbits, dogs, cats, horses, cattle, goat, sheep, pigs, monkeys, and humans can be mentioned. An example of the non-adhesive adult stem cells is momonuclear cells. Other examples of the non-adhesive adult stem cells are pluripotent stem cells, mesodermal stem cells, vascular/hematopoietic stem cells, hematopoietic stem cells, vascular stem cells, vascular endothelial progenitor cells, vascular endothelium-like progenitor cells, and monocytes. Still other examples of the non-adhesive adult stem cells are cells positive for 1 or more cell surface markers selected from the group consisting of CD14, CD31, CD34, CD133, CD90 (Thy-1) and ABCG-2.

The non-adhesive adult stem cells can also be cells derived from bone marrow, cord blood or peripheral blood. When cells derived from peripheral blood are used as the non-adhesive adult stem cells, the non-adhesive adult stem cells can be prepared from peripheral blood wherein non-adhesive adult stem cells are recruited by a substance capable of recruiting the non-adhesive adult stem cells. As examples of the substance capable of recruiting the non-adhesive adult stem cells, G-CSF, SDF-1, estrogen, VEGF, GM-CSF, angiopoietin 1 and 2, HGF, statins, and erythropoietin can be mentioned.

As used herein, "adhesive feeder cells" refer to adhesive cells that secrete or present a certain factor or ligand that aids the expansion of non-adhesive adult stem cells (for example, Notch ligand, Eph, Ephrin and the like), used in the expansion method of the present invention. As the animal species from which the adhesive feeder cells are derived, the same as the animal species from which the non-adhesive adult stem cells are derived can be mentioned. An example of the adhesive feeder cells is momonuclear cells. Other examples of the adhesive feeder cells are stromal cells, vascular endothelium-like cells, vascular smooth muscle-like cells, mesenchymal cells, myelocytes, monocytes, and macrophages (or cells derived therefrom). Still other examples of the adhesive feeder cells are cells positive for 1 or more cell surface markers selected from the group consisting of CD45, Delta-like-1, Delta-like-3, Delta-like-4, Jagged-1, Jagged-2, Eph, and Ephrin. The adhesive feeder cells can also be cells negative for CD45.

The adhesive feeder cells can also be cells derived from bone marrow, cord blood or peripheral blood. When cells derived from peripheral blood are used as the adhesive feeder cells, the adhesive feeder cells can be prepared from peripheral blood wherein the adhesive feeder cells are recruited by a substance capable of recruiting the adhesive feeder cells. As the substance capable of recruiting the adhesive feeder cells, the same as the substance capable of recruiting the non-adhesive adult stem cells can be mentioned.

As used herein, "non-adhesive non-stem cells" refer to non-adhesive cells that secrete or present a certain factor or ligand that aids the expansion of non-adhesive adult stem cells (for example, gp130(CD130), Delta-Like-1, Delta-like-3, Delta-like-4, Jagged-1, Jagged-2, SCF, Eph, Ephrin), used in the expansion method of the present invention. As the animal species from which the non-adhesive non-stem cells are derived, the same as the animal species from which the non-adhesive adult stem cells are derived can be mentioned. An example of the non-adhesive non-stem cells is momonuclear cells. Other examples of the non-adhesive non-stem cells are myelocytes, monocytes, macrophages, and dendritic cells (or cells derived therefrom). Still other examples of the non-adhesive non-stem cells are cells positive for 1 or more cell surface markers selected from the group consisting of CD45, CD33, CD11b, CD11c, HLA-ABC, and HLA-DR. The non-adhesive non-stem cell can also be cells negative for 1 or more cell surface markers selected from the group consisting of CD34, CD133, ABCG-2, CD31, CD14, and CD90.

The non-adhesive non-stem cells can also be cells derived from bone marrow, cord blood or peripheral blood. When cells derived from peripheral blood are used as the non-adhesive non-stem cells, the non-adhesive non-stem cells can be prepared from peripheral blood wherein the non-adhesive non-stem cells are recruited by a substance capable of recruiting the non-adhesive non-stem cells. As the substance capable of recruiting the non-adhesive non-stem cells, the same as the substance capable of recruiting the non-adhesive adult stem cells can be mentioned.

A feature of the expansion method of the present invention resides in that the ratio of non-adhesive adult stem cell count to total cell count (including non-adhesive adult stem cells, adhesive feeder cells, and non-adhesive non-stem cells) in the medium the time of start of cultivation is kept low. In a conventional cultivation method, the ratio of stem cell count to total cell count in the medium has been not less than 50%. However, in the cultivation method, the total cell count increases but the positive rate of the stem cells decreases, so that it has been impossible to efficiently expand stem cells per se. On the other hand, the present inventors succeeded in efficiently expanding stem cells by keeping the ratio of stem cell count to total cell count in the medium low to relatively increase the ratio of cells other than stem cells (including both non-adhesive cells and adhesive cells) in the medium. Although the ratio of stem cell count to total cell count in the medium is not subject to limitation, as long as it allows efficient expansion of stem cells, the ratio can be, for example, less than 40%, preferably not more than 30%, more preferably not more than 20%, still more preferably not more than 10%, most preferably not more than 5% or not more than 3%. When CD34⁺ cells and/or CD133⁺ cells are used as the non-adhesive adult stem cells, the ratio of CD34⁺ cell count and/or CD133⁺ cell count, respectively, to total cell count in the medium can be, for example, less than 40%, preferably not more than 30%, more preferably not more than 20%, still more preferably not more than 15%, most preferably not more than 10%, not more than 8% or not more than 5%. The ratio of non-adhesive adult stem cell count to total cell count in the medium at the time of start of cultivation can be calculated by, for example, measuring the non-adhesive adult stem cell count utilizing a cell surface marker for non-adhesive adult stem cells, and then dividing the count by the total cell count.

A ratio of non-adhesive adult stem cell count that enables efficient expansion can also be defined relative to total non-adhesive cell count. Although the ratio of stem cell count to total non-adhesive cell count in the medium is not subject to limitation, as long as it enables efficient expansion of stem cells, the ratio can be, for example, less than 50%, preferably not more than 40%, more preferably not more than 30%, still more preferably not more than 20%, most preferably not more than 10% or not more than 5%. When CD34⁺ cells and/or CD133⁺ cells are used as the non-adhesive adult stem cells, the ratio of CD34⁺ cell count and/or CD133⁺ cell count, respectively, to total non-adhesive cell count in the medium can be, for example, less than 50%, preferably not more than 40%, more preferably not more than 30%, still more preferably not more than 20%, most preferably not more than 10%, not more than 5% or not more than 3%. The ratio of non-adhesive adult stem cell count to total non-adhesive cell count in the medium can be calculated by, for example, collecting a portion of the culture broth after elapse of a sufficient time to achieve cell adhesion from the time of start of cultivation (for example, 1 or 2 weeks), measuring the non-adhesive adult stem cell count contained in the culture broth utilizing a cell surface marker therefor, and then dividing the count by the total cell count contained in the portion of the culture broth. It has been proven that according to the expansion method of the present invention, the ratio of non-adhesive adult stem cell count to total non-adhesive cell count in the medium is nearly constant throughout the cultivation period.

The expansion method of the present invention also comprises adding a biological sample (including non-adhesive adult stem cells, adhesive feeder cells and non-adhesive non-stem cells) collected from an animal to a serum-free medium without a cell fractionation treatment utilizing a cell surface marker, then culturing the non-adhesive adult stem cells in the co-presence of the adhesive feeder cells and the non-adhesive non-stem cells in the serum-free medium. Conventionally, in culturing non-adhesive adult stem cells such as bone-marrow momonuclear cells, a biological sample comprising the stem cells was collected from an animal, the stem cells were fractionated from this biological sample, and then the fraction was added to the medium and cultured therein. The reason why this complicated technique has been employed is that a focus has conventionally been placed on research into stem cells per se or research into the expansion of stem cells alone, so that extensive analysis has been facilitated by purifying them. That is, it has been a common practice of global standard to identify stem cell markers, and to study stem cell properties, expansion capability, possible applications and the like after purification of the stem cells. However, the present inventors have found that in biological samples such as bone marrow fluid, cord blood, and peripheral blood, non-adhesive adult stem cells, adhesive feeder cells and non-adhesive non-stem cells are present in a ratio suitable for efficient expansion of non-adhesive adult stem cells (i.e., a state wherein the ratio of non-adhesive adult stem cells to total cell count in the medium is kept low compared to conventional method), and hence that non-adhesive adult stem cells can be more efficiently expanded by adding a biological sample collected from an animal to a serum-free medium without any cell fractionation treatment utilizing a cell surface marker, and performing cultivation. As is also evident from the fact that it has become possible to more efficiently expand non-adhesive adult stem cells compared to the conventional method, despite its more convenient operation by the obviation of a prior cell fractionation treatment utilizing a cell surface marker (for example, FACS (fluorescence activated cell sorter), MACS (magnetic activated cell sorter)) compared to the conventional method, the expansion method of the present invention is very convenient and highly practical. The biological sample collected from an animal may be added directly (i.e., without being treated) to the serum-free medium, or may be added after a specified treatment. As examples of the operation in case where the biological sample collected from an animal is treated before being added to the serum-free medium, centrifugation methods such as specific density centrifugation (using, for example, Histopac, Ficoll and the like) and convenient operations such as hemolysis methods (using, for example, NH₄Cl, hypotensive solution and the like) can be mentioned. When adding the biological sample collected from an animal to the serum-free medium, the count of cells inoculated to the medium is not subject to limitation, as long as it produces a cell density suitable for the expansion of non-adhesive adult stem cells; cells are inoculated to the medium so that the total cell count in the medium (i.e., count of cells including non-adhesive adult stem cells, adhesive feeder cells and non-adhesive non-stem cells) will be, for example, about 0.5x10⁵ to 10x10⁵ cells/ml, preferably about 1x10⁵ to 5x10⁵ cells/ml.

When the biological sample collected from an animal is added to the serum-free medium and cultured without any cell fractionation treatment, it is also possible to obtain cells suitable for syngenic transplantation by using a sample derived from an individual for which cell transplantation is intended as the biological sample. Therefore, expanded cells obtained by the expansion method of the present invention or differentiated cells thereof have the advantage of avoiding risks for infection and graft rejection during cell transplantation.

The expansion method of the present invention can further comprise adjusting the ratio of non-adhesive adult stem cells to total cell count or total non-adhesive cell count before the start of cultivation. The present inventors found that the ratio of non-adhesive adult stem cell count to total cell count (or total non-adhesive cell count) in the medium was very important to the expansion efficiency for the stem cells. Therefore, in adjusting the ratio of non-adhesive adult stem cells, it is preferable that the ratio of the stem cells be reduced. The ratio to be reached can be, for example, the above-described ratio that enables efficient expansion of non-adhesive adult stem cells.

An adjustment of the ratio of non-adhesive adult stem cells can be performed by a method known per se. For example, the adjustment can be performed by adding non-adhesive adult stem cells and non-stem cells (adhesive feeder cells, non-adhesive non-stem cells) in an appropriate ratio to a serum-free medium after their isolation/purification (having the same definition as fractionation), and culturing the non-adhesive adult stem cells and the adhesive feeder cells. Isolation/purification of the cells can be performed by a method known per se utilizing a cell surface marker, for example, FACS or MACS. Also, if the non-adhesive adult stem cells, adhesive feeder cells, and non-adhesive non-stem cells are separately isolated/purified, desired cells (for example, non-adhesive adult stem cells) selected from among the non-adhesive adult stem cells, adhesive feeder cells, and non-adhesive non-stem cells may be subjected to a particular treatment (for example, marking treatment).

In a method utilizing a cell surface marker, a substance having specific affinity to the cell surface marker is used. As such substance, for example, an antibody having specific affinity to these proteins or a fragment thereof can be mentioned. The specific affinity means the ability to specifically recognize and bind to the protein by antigen-antibody reaction. The antibody or fragment thereof is not particularly limited as long as it can specifically bind to the protein. It may be a polyclonal antibody, monoclonal antibody or functional fragment thereof. These antibody or functional fragment thereof can be produced by methods generally used in the art. For example, when a polyclonal antibody is used, a method comprising injecting the protein subcutaneously on the back, intraperitoneally, intravenously or the like to an animal such as mouse and rabbit to immunize the animal and, after increase of the antibody titer, collecting the antiserum can be mentioned. When a monoclonal antibody is used, a method comprising producing hybridoma according to a conventional method and collecting a secretory fluid thereof can be mentioned. As a method of producing an antibody fragment, a method comprising expression of a cloned antibody gene fragment in a microorganism and the like is frequently used. The purity of the antibody, antibody fragment and the like is not particularly limited as long as it maintains specific affinity to the protein. The antibody and fragment thereof may be labeled with a fluorescent substance, enzyme, radioisotope or the like.

In adjusting the ratio of non-adhesive adult stem cells, the non-adhesive adult stem cells, adhesive feeder cells and non-adhesive non-stem cells used for the cultivation can be those derived from the same animal species. By using cells derived from an individual for which cell transplantation is intended as the non-adhesive adult stem cells, adhesive feeder cells and non-adhesive non-stem cells, it is also possible to obtain cells suitable for syngenic transplantation. Therefore, expanded cells obtained by the expansion method of the present invention or differentiated cells thereof have the advantage of avoiding risks for infection and graft rejection during cell transplantation.

Another feature of the expansion method of the present invention resides in that a serum-free medium of a distinct composition, that is, a chemically defined serum-free medium, is used in the expansion of non-adhesive adult stem cells. Therefore, the expansion method of the present invention has the advantages of excellence in quality control and validation, and of the capability of being clinically applied and industrialized, or preference therefor. The serum-free medium used in the expansion method of the present invention is hereinafter described in detail.

As the basal medium used for the serum-free culture medium of the present invention, any medium generally used in the art can be utilized. For example, a serum-free culture medium known as a medium for growth of hematopoietic stem cells can be used. As the basal medium used as the serum-free culture medium, for example, DMEM, α-MEM, IMDM, RPMI1640 and the like can be mentioned, with preference given to IMDM.

The serum-free medium of the present invention can comprise, for example, interleukin 3 and/or a fatty acid.

Interleukin 3 (IL-3) is known as a cytokine that acts on hematopoietic stem cells and progenitor cells of various blood cell lineage to promote their growth and differentiation. The IL-3 used in the present invention is chosen as appropriate; when human non-adhesive adult stem cells are expanded, human IL-3 is preferable. From the viewpoint of quality control and validation, a recombinant is preferable. The concentration of IL-3 in the serum-free medium varies also depending on the type of IL-3 used, and is not subject to limitation, as long as it enables efficient expansion of non-adhesive adult stem cells; when human recombinant IL-3 is used, the concentration is about 5 to 500 ng/mL, preferably about 10 to 200 ng/mL, more preferably about 50 ng/mL.

As the fatty acid, saturated or unsaturated fatty acids or a mixture thereof can be used. The fatty acid is a nutritional factor lacked in the conventional media used for cultivation of non-adhesive adult stem cells. The present inventors found that this factor was important for expanding non-adhesive adult stem cells ex vivo for a long time while maintaining their pluripotency and self-replication capability. As examples of the fatty acid, arachidonic acid, linoleic acid, linolenic acid, myristic acid, oleic acid, palmitoleic acid, palmitic acid, and stearic acid can be mentioned. The concentration of fatty acid in the serum-free medium varies also depending on the type of fatty acid used, and is not subject to limitation, as long as it enables more efficient expansion of non-adhesive adult stem cells; the concentration is, for example, about 1 to 1000 ng/ml, preferably about 5 to 500 ng/ml, more preferably about 10 to 200 ng/ml.

The serum-free medium of the present invention can also comprise 1 or 2 or more factors, preferably 3 or more factors, more preferably 4 or more factors, still more preferably 5 or more factors, most preferably 6 or more or all factors, selected from the group consisting of Flk2/Flt3 ligand, stem cell factor, thrombopoietin, interleukin 6, insulin, transferrin and albumin.

The Flk2/Flt3 ligand (FL) is a ligand for the tyrosine kinase receptor Flk2/Flt3, which is known to transmit a signal important for the development of hematopoietic stem cells. The FL used in the present invention is chosen as appropriate; when human non-adhesive adult stem cells are expanded, human FL is preferable. From the viewpoint of quality control and validation, a recombinant is preferable. The concentration of FL in the serum-free medium varies depending on the type of FL used, and is not subject to limitation, as long as it enables more efficient expansion of non-adhesive adult stem cells; when human recombinant FL is used, the concentration is, for example, about 5 to 500 ng/mL, preferably about 10 to 200 ng/mL, more preferably about 50 ng/mL.

The stem cell factor (SCF) to be used in the present invention is a glycoprotein with about 30,000 of molecular weight, consisting of 248 amino acids. While there exist a soluble form and a membrane-bound form due to alternative splicing. It is preferably of a soluble form. The SCF used in the present invention is chosen as appropriate; when human non-adhesive adult stem cells are expanded, human SCF is preferable. From the viewpoint of quality control and validation, a recombinant is preferable. The concentration of SCF in the serum-free culture medium varies depending on the kind of SCF to be used, and is not subject to limitation, as long as it enables more efficient expansion of non-adhesive adult stem cells. When human recombinant SCF is used, the concentration is, for example, about 5 to 500 ng/mL, preferably about 10 to 200 ng/mL, more preferably about 50 ng/mL.

The thrombopoietin (TPO) used in the present invention is a kind of hematopoietic cytokine, and is known to act specifically on the process of megakaryocyte formation from hematopoietic stem cells to promote the production of megakaryocytes. The TPO used in the present invention is chosen as appropriate; when human non-adhesive adult stem cells are expanded, human TPO is preferable. From the viewpoint of quality control and validation, a recombinant is preferable. The concentration of TPO in the serum-free medium varies also depending on the type of TPO used, and is not subject to limitation, as long as it enables more efficient expansion of non-adhesive adult stem cells; when human recombinant TPO is used, the concentration is, for example, about 5 to 500 ng/mL, preferably about 10 to 200 ng/mL, more preferably about 50 ng/mL.

Interleukin-6 (IL-6) to be used in the present invention is a glycoprotein with 210,000 of molecular weight, isolated as a factor introducing terminal differentiation of a B cell into an antibody-producing cell, and known to be involved in immune response, expansion and differentiation of a hematopoietic or neural cell, acute-phase reaction and the like. While IL-6 to be used in the present invention can be appropriately selected, when it is used for expanding human non-adhesive adult stem cells, human IL-6 is preferable. In addition, a recombinant is preferable from the aspects of quality control and validation. The concentration of IL-6 in the serum-free culture medium varies depending on the kind of IL-6 to be used, and is not particularly limited as long as it enables more efficient expansion of non-adhesive adult stem cells. When human recombinant IL-6 is used, the concentration is, for example, about 5 - 500 ng/mL, preferably about 10 - 200 ng/mL, more preferably about 50 ng/mL.

The insulin used in the present invention is chosen as appropriate; when human non-adhesive adult stem cells are expanded, human insulin is preferable. From the viewpoint of quality control and validation, a recombinant is preferable. The concentration of insulin in the serum-free medium varies also depending on the type of insulin used, and it is not subject to limitation, as long as it enables more efficient expansion of non-adhesive adult stem cells; when human recombinant insulin is used, the concentration is, for example, about 0.5 to 100 µg/mL, preferably about 2 to 50 µg/mL, more preferably about 10 µg/mL.

The transferrin used in the present invention is chosen as appropriate; when human non-adhesive adult stem cells are expanded, human transferrin is preferable. From the viewpoint of quality control and validation, a recombinant is preferable. The concentration of transferrin in the serum-free medium varies also depending on the type of transferrin used, and is not subject to limitation, as long as it enables more efficient expansion of non-adhesive adult stem cells; when human recombinant transferrin is used, the concentration is, for example, about 5 to 5000 µg/mL, preferably about 50 to 1000 µg/mL, more preferably about 200 µg/mL.

The albumin used in the present invention is chosen as appropriate from among desired albumins (for example, bovine serum albumin, human albumin); when human non-adhesive adult stem cells are expanded, human albumin is preferable. From the viewpoint of quality control and validation, a recombinant is preferable. The concentration of albumin in the serum-free medium varies also depending on the type of albumin used, and is not subject to limitation, as long as it enables more efficient expansion of non-adhesive adult stem cells; when human recombinant albumin is used, the concentration is, for example, about 0.5 to 100 mg/mL, preferably about 2 to 50 mg/mL, more preferably about 10 mg/mL.

Regarding the factor added to the serum-free medium, used in the present invention, it is preferable that the animal species from which it is derived be uniformized to the animal species from which the non-adhesive adult stem cells and/or adhesive feeder cells are derived. Uniformizing the derivation of the animal species has the advantage of avoiding graft rejection reactions during transplantation of expanded cells or differentiated cells thereof.

The serum-free medium of the present invention may further be supplemented with ingredients such as amino acids, vitamins (for example, vitamin E), lipids (for example, cholesterol, in addition to the foregoing fatty acids), antibiotics, buffering agents, and surfactants (for example, PLURONIC, TWEEN). The pH of the medium is, for example, about 6 to 8, preferably about 6.5 to 7.5. Cultivation temperature is, for example, about 30 to 40°C, preferably about 37°C.

Each of the above-mentioned components is dissolved in a serum-free culture medium to a given concentration, or a concentrated solution of each component (stock solution) is prepared in advance and diluted with a serum-free culture medium to a given concentration, whereby the serum-free culture medium of the present invention can be prepared. For example, the serum-free culture medium of the present invention can be prepared by dissolving the necessary components in a commercially available serum-free culture medium to given concentrations and sterilizing the medium by filtration and the like, or aseptically adding the stock solutions sterilized by filtration and the like to a commercially available serum-free culture medium to dilute them. Sterilization by filtration can be performed according to a method generally employed in the art. For example, it is performed using 0.22 µm or 0.45 µm of Millipore filter and the like.

The culture vessel used in the expansion method of the present invention is not subject to limitation; from the viewpoint of efficient adhesion of adhesive feeder cells, a cell adhesive culture vessel is preferable. For example, an culture vessel coated with a substance that aids cell adhesion, such as an extracellular matrix (for example, poly-D-lysine, laminin, fibronectin), may be used. As the culture vessel, flasks, dishes, plates, chamber slides, Petri dishes, tubes, trays, culture bags, bottles and the like can also be used.

According to the cultivation method of the present invention, to more efficiently expand non-adhesive adult stem cells, it is also preferable that settings be made to allow the cells to cluster, and/or that the amount of medium per unit number of cells be sufficient. The reason why clustering of the cells is preferred is that intercellular contact is important for more efficient expansion of non-adhesive adult stem cells; the reason why it is preferable that the amount of medium per unit number of cells be sufficient is that a lack of medium ingredients, additive factors and the like is prevented, and that the concentrations of factors secreted from the cells are prevented from increasing in excess. The degree of clustering of the cells is not subject to limitation, as long as it enables more efficient expansion of non-adhesive adult stem cells; the cells can be cultured so that a large number of cells inoculated to the medium, for example, not less than 70%, preferably not less than 80%, more preferably not less than 85%, still more preferably not less than 90%, most preferably not less than 95%, of the cells will cluster in a low volume, for example, 3/10 volume, preferably 2.5/10 volume, more preferably 2/10 volume, still more preferably 1.5/10 volume, most preferably 1/10 volume, of the medium. The amount of medium per unit number of cells is also not subject to limitation, as long as it enables more efficient expansion of non-adhesive adult stem cells; for example, a preferable amount of medium is not less than about 0.1 ml/1.0x10⁵ cells, preferably not less than about 0.3 ml/1.0x10⁵ cells, more preferably not less than about 0.5 ml/1.0x10⁵ cells, still more preferably not less than about 1 ml/1.0x10⁵ cells, most preferably not less than about 2 ml/1.0x10⁵ cells. In a 6-well plate (each well having a diameter of 35 mm), because cells spontaneously cluster at the centers of the wells, these conditions are very easy to achieve. However, because those skilled in the art are able to set similar conditions as appropriate for other culture vessels, other culture vessels can also be preferably used. In the case of other culture vessels, these conditions can more easily be achieved by using a membrane that allows the passage of ingredients and factors in the medium, and that does not allow the passage of cells (for example, having a pore size of about 0.1 to 1 µm, preferably about 0.4 µm). As examples of the membrane, Culture Insert can be mentioned.

Cultivation period can be adjusted as appropriate so that a sufficient amount of non-adhesive adult stem cells is expanded. Referring to cell counts, expansion by about 20 fold is expected after 2 weeks, about 100 fold after 4 weeks, and about 500 fold after 6 weeks and it varies also depending on the type of non-adhesive adult stem cells and the like;. According to the cultivation method of the present invention, it has been confirmed that by extending the cultivation period and the like, non-adhesive adult stem cells are expanded about 100,000,000 fold.

The expansion method of the present invention can further comprise confirming expansion (for example, expansion rate) of non-adhesive adult stem cells. Confirmation of the expansion can be performed by comparing cell counts having a specified cell surface marker between before and after the expansion. A measurement of a cell count having a specified cell surface marker can be performed by a method known per se, such as FACS.

The expansion method of the present invention can still further comprise isolating/purifying the non-adhesive adult stem cells expanded. Isolation/purification of the expanded cells can be performed by a method known per se, for example, a cell sorting method utilizing a cell surface marker (see, for example, the above-described method).

The expansion method of the present invention can further comprise differentiating the non-adhesive adult stem cells expanded to obtain differentiated cells (further comprise isolating/purifying the cells as required). Because the non-adhesive adult stem cells expanded by the expansion method of the present invention have pluripotency and self-replication capability, they can be differentiated into various cells. Cells into which the non-adhesive adult stem cells expanded by the expansion method of the present invention are capable of differentiating are not subject to limitation; for example, vascular endothelial cells, vascular smooth muscle cells, lymphatic endothelial cells, skeletal muscle cells, myocardial cells, neural lineage cells, blood lineage cells, hepatocytes, pancreas cells, kidney cells, and epithelial cells (for example, intestinal epithelial cells, dermal epithelial cells) can be mentioned. These cells can be obtained by, for example, culturing in vitro under conditions suitable for inducing the differentiation using various induction factors, or in vivo. Such methods are commonly known; for example, as examples of induction factors for obtaining vascular endothelial cells, VEGF-A, FGF, and HGF can be mentioned; as examples of induction factors for obtaining vascular smooth muscle cells, PDGF can be mentioned; as examples of induction factors for obtaining lymphatic endothelial cells, VEGF-C and VEGF-D can be mentioned; as examples of induction factors for obtaining skeletal muscle cells, IGF-I and IGF-II can be mentioned; as an example of the method for obtaining myocardial cells, co-cultivation with myocardial cells can be mentioned; as examples of induction factors for obtaining neural lineage cells, NGF, BDNF, GDNF, NT-3, NT-4, and NT-5 can be mentioned; as examples of induction factors for obtaining hematopoietic stem cells, SCF, TPO, FL, IL-3, and IL-6 can be mentioned; as examples of induction factors for obtaining hepatocytes, HGF and EGF can be mentioned; as examples of induction factors for obtaining pancreas cells, HGF, FGF, and EGF can be mentioned; as examples of induction factors for obtaining kidney cells, HGF, FGF, and EGF can be mentioned; as examples of induction factors for obtaining epithelial cells, KGF, EGF, and FGF can be mentioned. Differentiated cells can be isolated/purified as required. Isolation/purification of differentiated cells can be performed by a method known per se, for example, a cell sorting method utilizing a cell surface marker (see, for example, the above-described method).

The present invention also provides a cell obtainable by the method of the present invention. As the cell obtainable by the method of the present invention, for example, non-adhesive adult stem cells and differentiated cells thereof can be mentioned.

The present invention further provides a composition comprising the cell of the present invention, for example, a pharmaceutical composition. By unifying the derivation of non-adhesive adult stem cell, adhesive feeder cell and various factors to be used in the expansion method of the present invention, a composition substantially free of a biological component derived from a different animal species can be obtained. As used herein, the composition "substantially" free of a biological component derived from a different animal species means the quality affordable by culturing cells without using a cell and a biological component from a different animal species. In addition, using a non-adhesive adult stem cell and a cell derived from an individual who intends to undergo a cell transplantation as an adhesive feeder cell, a composition suitable for syngeneic transplantation can also be obtained. Accordingly, the cell of the present invention is advantageous in that a risk of infection and rejection on transplantation and the like can be avoided. The composition of the present invention can contain a pharmaceutically acceptable carrier.

The non-adhesive adult stem cell and a differentiated cell thereof obtainable by the method of the present invention can be used for the treatment of various diseases. For example, the non-adhesive adult stem cell as well as vascular endothelial cell and vascular smooth muscle cell, which are differentiated cells thereof, can be used for the treatment of vascular disorders by way of cell transplantation. As such vascular disorder, for example, ischemic diseases (e.g., ischemic cardiac diseases, Burger disease, arteriosclerosis obliterans (ASO)), vascular injury, post-PTCA restenosis and in-stent stenosis can be mentioned. It can also be used for curing a wound such as skin ulcer and the like or for producing an artificial blood vessel. The effect after cell transplantation can be confirmed by a method known per se. For example, when the vascular disorder is an ischemic cardiac disease, the posttransplant cardiac function can be evaluated, for example, by determining the contractile function and diastolic function. The contractile function or diastolic function of the heart can be determined by various methods used in the art. For example, the contractile function can be determined by measuring +dp/dt value (which decreases as the contractile function decreases) calculated from the mitral regurgitation waveform or left ventricular ejection fraction (%EF, which decreases as the contractile function decreases) and the like. The diastolic function can be similarly determined by measuring -dp/dt value (which decreases as the diastolic function decreases) calculated from mitral regurgitation waveform or end-diastolic inner diameter (EDd) and the like (e.g., see FASEB Journal, 18:1392-1394 (2004)).

The present invention further provides a serum-free medium comprising the above-described factors (for example, IL-3, fatty acid), and a reagent for culturing non-adhesive adult stem cells, comprising this serum-free medium. The serum-free medium of the present invention may also comprise adhesive feeder cells.

The present invention also provides a kit comprising the above-described factors (for example, IL-3, fatty acid) and a serum-free medium. The kit of the present invention is provided in a form wherein at least one factor of the above-described factors is separated from the serum-free medium (for example, placed in a different container). The kit of the present invention may further comprise adhesive feeder cells. The adhesive feeder cells are provided in a form present in the serum-free medium, or in a form separated from the serum-free medium. The kit of the present invention is useful for, for example, preparation of the serum-free medium of the present invention.

The kit of the present invention may further contain a substance (e.g., antibody) having specific affinity to the cell surface marker for a non-adhesive adult stem cell or differentiated cell thereof, and/or a differentiation-inducing factor for the non-adhesive adult stem cell. Such kit is preferably used in the method of the present invention.

The present invention is explained in more detail in the following by referring to the Examples, which are described for explanation of the present invention and do not limit the present invention in any way.

### Examples

### Example 1: Time-course changes in expansion rate for each cell population

### (1) Preparation of the serum-free medium of the present invention

A medium for in vitro expanding cultivation of stem cells was prepared by adding, to Iscov modified Dulbecco medium (Gibco 12440-053), 10 mg/ml bovine serum albumin, 10 µg/ml human recombinant insulin, 200 µg/ml human non-recombinant (that is, plasma-derived) transferrin, 50 ng/ml human recombinant FL, 50 ng/ml human recombinant SCF, 50 ng/ml human recombinant TPO, 50 ng/ml human recombinant IL-3, 50 ng/ml human recombinant IL-6, 50 µg/ml gentamycin, fatty acids and the like (20 ng/ml arachidonic acid, 2.2 µg/ml cholesterol, 700 ng/ml vitamin E (DL-α-tocopherol acetate), 100 ng/ml linoleic acid, 100 ng/ml linolenic acid, 100 ng/ml myristic acid, 100 ng/ml oleic acid, 100 ng/ml palmitoleic acid, 100 ng/ml palmitic acid, 100 ng/ml stearic acid, 1 mg/ml PLURONIC F-68, 22 µg/ml Tween 80).

### (2) In vitro expansion of cells

First, 15 ml of bone marrow fluid was collected from a human. The 15 ml of bone marrow fluid collected was mixed with 15 ml of PBS, overlaid on 15 ml of HISTOPAQUE-1077 (Sigma) in a 50 ml tube, and subjected to specific gravity centrifugation (600xg, 30 minutes, 20°C). Next, the uppermost layer (plasma, platelets, PBS) was aspirated and discarded, the layer containing momonuclear cells (the layer above HISTOPAQUE) was recovered in another tube, 10 ml of PBS-2 mM EDTA for washing was added thereto, and they were mixed and centrifuged at 1000xg for 20 minutes at 4°C, after which the supernatant was discarded, and the pellets were re-suspended. To the pellets obtained, 20 ml of NH₄Cl for hemolysis was added; the cells were incubated for 5 minutes and centrifuged at 370xg for 8 minutes at 4°C, after which the supernatant was discarded, and the pellets were re-suspended. To the pellets obtained, 10 ml of PBS for washing was added; the mixture was centrifuged at 200xg for 8 minutes at 4°C, after which the supernatant (including platelets) was discarded, whereby a fraction comprising bone-marrow momonuclear cells was obtained at a purity of not less than 80 to 90%.

The fraction prepared as described above was suspended in the medium for in vitro expansion of stem cells prepared as described above to obtain a cell density of 1x10⁵ cells/ml, and this suspension was then inoculated to a 6-well plate (each well having a diameter of 35 mm), pretreated for tissue culture, to obtain a cell density of 2x10⁵ cells/2 ml per well. Cultivation was performed under the conditions of 37°C, 5% CO₂, 20% O₂ for 2, 4 or 6 weeks. The medium was exchanged with a fresh supply with 1/2 dilution in the same well every week. In this cultivation, the positive rates of CD34⁺ cells and CD133⁺ cells (non-adhesive adult stem cells) to the count of total cells in the medium at the time of start of cultivation were 3.9% and 7.5%, respectively. Because some of the bone-marrow momonuclear cells inoculated in the medium adhered to the 6-well plate, and also because adhesive cells were present throughout the cultivation period, they were considered to be functioning as feeder cells. Furthermore, throughout the cultivation period, cells (including non-adhesive adult stem cells, adhesive feeder cells, and non-adhesive non-stem cells) clustered at the centers of the wells of the 6-well plate, and a state of locally increased cell density was maintained (not less than 95% of the cells inoculated to the medium were present in 1/10 volume of the medium).

At 2, 4 or 6 weeks after the start of cultivation, the individual types of cells were counted by FACS utilizing cell surface markers, and the expansion fold was calculated for each cell type. The cell surface markers for the respective cells are as follows: vascular stem cells (CD34⁺); vascular/hematopoietic stem cells (CD133⁺); vascular endothelial-like progenitor cells (CD14⁺); hematopoietic stem cells (CD34⁺, CD38⁻). Relative to each cell count at the time of start of cultivation (0) as 1, cell counts were measured at 2 weeks (2), at 4 weeks (4), and at 6 weeks (6) after the start of cultivation, to obtain expansion fold.

The results are shown in Table 1.

**Table 1**

| | 0 | 2 | 4 | 6 |
|---|---|---|---|---|
| Total non-adhesive cells | 1 | 20 | 100 | 500 |
| Vascular stem cells (CD34+) | 1 | 27 | 177 | 756 |
| Vascular/hematopoietic stem cells (CD133+) | 1, | 23 | 95 | 530 |
| Undifferentiated vascular endothelial-like cells (CD14+) | 1 | 29 | 167 | 939 |
| Hematopoietic stem cells (CD34+CD38-) | 1 | 34 | 285 | 1150 |
| Bone marrow fluid required for transplantation (mL) | 500 | ≤25 | ≤5 | ≤1 |

The volume of bone marrow fluid required, 500 mL when using cells before expanding cultivation for transplantation of each cell type, became not more than 25 mL after 2 weeks, not more than 5 mL after 4 weeks, not more than 1 mL after 6 weeks; after 6 weeks of expanding cultivation, the cells were expanded not less than 500 fold, and the volume of the bone marrow fluid required became 1/500, that is, 1 ml.

When expanding cultivation of bone-marrow momonuclear cells was continued in the same manner as described above, and the expansion efficiency of the expanded non-adhesive adult stem cells (including vascular stem cells, vascular/hematopoietic stem cells and the like) was calculated, the cell was expanded 2,000 fold at 8 weeks after expanding cultivation, 8,000 fold at 10 weeks, 32,000 fold at 12 weeks, 130,000 fold at 14 weeks, 500,000 fold at 16 weeks, 2,000,000 fold at 18 weeks, 8,000,000 fold at 20 weeks, 32,000,000 fold at 22 weeks, and 100,000,000 fold or more at 24 weeks.

It was confirmed that when CD34⁺ cells purified to about 90% were cultured in the same manner as described above, the total cell count became 30 fold but the CD34⁺ positive rate decreased to not more than 50% after 1 week of cultivation, and that when CD34⁺ cells purified to about 40% were cultured in the same manner as described above, the total cell count became 30 fold but the CD34⁺ positive rate decreased to not more than 2% after 1 week of cultivation. It was also confirmed that when CD34⁺ cells purified to about 90% or 40% were cultured in the same manner as described above, the CD34⁺ positive rate became not more than 1% after 1 week of cultivation. As stated above, the positive rate becomes nearly 0% after 2 weeks of cultivation; therefore, even if the total expansion rate is 500 fold, the expansion rate for stem cells per se is up to about 0 to 10 fold. Therefore, it was shown that by keeping the ratio of non-adhesive adult stem cells to total cell count (total non-adhesive cells) low, the expansion efficiency for the stem cells improved remarkably.

### Example 2: Temporal changes in each cell population

Furthermore, in the same manner as Example 1, cells derived from bone-marrow momonuclear cells were subjected to expanding cultivation, and the relative abundance of each cell population was examined at the time of start of cultivation (0), after 2 weeks of cultivation (2), after 4 weeks of cultivation (4), and after 6 weeks of cultivation (6). Cells were identified with CD31 positivity as the criterion for stem cell-rich cells, with CD45 negativity as the criterion for fibroblasts/bone stem cell group, and with both CD45 positivity and CD33 negativity as the criterion for the cell population, including lymphocytes.

The relative abundance of each cell population to total cell count is shown by %. The results are shown in Table 2.

**Table 2**

| | 0 | 2 | 4 | 6 |
|---|---|---|---|---|
| Vascular lineage stem cell-rich cells (CD31+) | 65.14 | 81.87 | 91.26 | 93.27 |
| Fibroblast/bone stem cell group (CD45-) | 25.55 | 0.02 | 0.09 | 0.04 |
| Cell population, including lymphocytes (CD45+CD33-) | 57.8 | 8.3 | 4.43 | 6.17 |

After 6 weeks of cultivation, there were almost no CD45-negative cells, and not less than 90% were CD31-positive. This means that the cultivation method of the present invention is capable of specifically expanding vascular lineage stem cells.

### Example 3: Transplantation of expanded stem cells to ischemic animal model

Expanded bone-marrow momonuclear cells (1x10⁶ cells), including stem cells, after expanding cultivation for 6 weeks by the method of the present invention in the same manner as Example 1, were used for transplantation to lower limb ischemia animals. The lower limb ischemia animal model was prepared by incising a skin of an immunodeficient rat (F344/N Jcl-rnu, 9 weeks of age, male) under anesthesia, anastomosing the femoral artery and vein, resecting the distal side, and suturing the skin. To the thigh and crus of this animal model, expanded bone-marrow momonuclear cells, including stem cells, were intramuscularly injected. At 3 weeks after cell transplantation, a tissue section of the tibialis anterior muscle was prepared, and stained with anti-HLA-class I antigen antibody (human cell specific), anti-α-SMC antibody (vascular smooth muscle cell specific), and anti-KDR antibody (human vascular endothelial cell specific).

As a result, vascular images co-stained by anti-HLA-class I antibody and anti-α-SMC antibody, or by anti-HLA-class I antibody and anti-KDR antibody, were confirmed, and it was confirmed that blood vessels were formed from the expanded cells transplanted. Also observed in the lower limb ischemia was an improvement of bloodstream.

### Example 4: Transplantation of expanded stem cells to animal model of muscular injury

Expanded bone-marrow momonuclear cells (6x10⁵ to 3x10⁶ cells), including stem cells, after expanding cultivation for 6 weeks by the method of the present invention in the same manner as Example 1, were used for transplantation to animal model of a tibialis anterior muscle injury. The animal model of tibialis anterior muscle injury was prepared by incising skin and fascia of an immunodeficient rat (F344/N Jcl-rnu, 6 to 7 weeks of age, male), and removing part of the tibialis anterior muscle using tweezers. To this site from which the part of the tibialis anterior muscle was removed, the expanded bone-marrow momonuclear cells, including stem cells, were transplanted, and then the fascia was sutured with the skin. At 4 weeks after cell transplantation, a tissue section of the tibialis anterior muscle was prepared, and stained with anti-HLA-class I antigen antibody (human cell specific) and anti-α-SMC antibody (vascular smooth muscle cells specific).

As a result, vascular images co-stained by anti-HLA-class I antibody and anti-α-SMC antibody were confirmed, and it was confirmed that blood vessels were formed from the expanded cells transplanted. The bloodstream in the injured tibial muscle was also normalized.

### Industrial Applicability

By the method of the present invention, it is possible to expand non-adhesive adult stem cells ex vivo at least 500 fold, and the expanded cells obtained can be induced to differentiate into various cells in vivo and in vitro. Furthermore, the expanded cells obtained were effective in improving bloodstream in an ischemia model and a muscular injury model. That is, by the method of the present invention, which enables the obtaining large amounts of adult stem cells, it is possible to provide a therapeutic method with a decreased burden on the patient for reportedly about 100,000 lower limb ischemia patients, including latent patients, and reportedly about 1,100,000 ischemic heart disease patients. Also, compared with the existing method, which comprises collecting not less than 500 ml of bone marrow fluid under general anesthesia, the method of the present invention requires only about 1 ml of bone marrow fluid collected under local anesthesia, so that the burden on the patient can be remarkably lessened. Furthermore, because the cells after expansion can be cryopreserved, repeated treatment, dosing of 1 lot to a plurality of patients and the like are also possible.

This application is based on a patent application No. 2005-052944 filed in Japan (filing date: February 28, 2005), the contents of which are incorporated in full herein by this reference.

## Claims

1. A method of expanding non-adhesive adult stem cells, comprising culturing the non-adhesive adult stem cells in the co-presence of adhesive feeder cells and non-adhesive non-stem cells in serum-free medium, wherein the ratio of non-adhesive adult stem cell count to total non-adhesive cell count in the medium is not more than 40%.

2. The method of claim 1, comprising adding a biological sample comprising non-adhesive adult stem cells, adhesive feeder cells and non-adhesive non-stem cells to serum-free medium without a cell fractionation treatment utilizing a cell surface marker, and then culturing the non-adhesive adult stem cells in the serum-free medium in the co-presence of the adhesive feeder cells and the non-adhesive non-stem cells.

3. The method of claim 1, further comprising adjusting the ratio of non-adhesive adult stem cells to total non-adhesive cell count before the start of cultivation.

4. The method of claim 1, further comprising confirming the expansion of non-adhesive adult stem cells, or isolating the non-adhesive adult stem cells expanded.

5. The method of claim 1, wherein the ratio of non-adhesive adult stem cell count to total non-adhesive cell count in the medium is not more than 20%.

6. The method of claim 1, wherein the non-adhesive adult stem cells comprise CD34 and/or CD133 positive cells.

7. The method of claim 1, wherein the non-adhesive adult stem cells are momonuclear cells.

8. The method of claim 1, wherein the adhesive feeder cells are momonuclear cells.

9. The method of claim 1, wherein the non-adhesive adult stem cells and/or adhesive feeder cells are derived from bone marrow, cord blood or peripheral blood.

10. The method of claim 1, wherein the non-adhesive adult stem cells and adhesive feeder cells are derived from the same animal species.

11. The method of claim 2, wherein the biological sample is selected from the group consisting of bone marrow fluid, cord blood and peripheral blood.

12. The method of claim 2, wherein the biological sample is derived from a human.

13. The method of claim 1, wherein the serum-free medium comprises interleukin 3 and a fatty acid.

14. The method of claim 1, further comprising differentiating non-adhesive adult stem cells expanded by culturing non-adhesive adult stem cells to obtain differentiated cells.

15. The method of claim 14, wherein the differentiated cells are selected from the group consisting of vascular endothelial cells, vascular smooth muscle cells, lymphatic endothelial cells, skeletal muscle cells, myocardial cell, neural lineage cells, blood lineage cells, hepatocytes, pancreas cells, kidney cells and epithelial cells.

16. The non-adhesive adult stem cells obtainable by the method of claim 1.

17. A composition comprising non-adhesive adult stem cells, and being substantially free from any biological ingredient derived from an animal heterologous to the animal from which the non-adhesive adult stem cells are derived.

18. A differentiated cell of non-adhesive adult stem cells, obtainable by the method of claim 14.

19. A serum-free medium comprising interleukin 3 and a fatty acid.

20. A kit for preparing a serum-free medium comprising interleukin 3 and a fatty acid, comprising interleukin 3, the fatty acid and a serum-free medium (one or both of interleukin 3 and the fatty acid are not added to the serum-free medium).
